# FASCICULE DE BREVET EUROPEEN

(11) **EP 2 376 409 B1**
(45) Date de publication et mention de la délivrance du brevet: **18.07.2018**
(21) Numéro de dépôt: 10706296.0
(22) Date de dépôt: 12.01.2010
(51) Int. Cl.: C07C 17/25, C07C 17/38, C07C 21/18, C07C 17/02

(54) **PROCEDE DE PREPARATION DE COMPOSES FLUORES OLEFINIQUES**
VERFAHREN ZUR HERSTELLUNG VON FLUOROLEFINVERBINDUNGEN
METHOD FOR THE PREPARATION OF FLUOROOLEFIN COMPOUNDS

(30) Priorité: 13.01.2009 FR 0950157
(43) Date de publication de la demande: 19.10.2011
(73) Titulaire: Arkema France, 92700 Colombes (FR)
(72) Inventeur: SEDAT, Pierre-Marie, 69210 Fleurieux sur l'Arbresle (FR); BOSSOUTROT, Jean-Michel, 69630 Chaponost (FR)
(74) Mandataire: Dang, Doris
(86) Numéro de dépôt international: PCT/FR2010/050043
(87) Numéro de publication internationale: WO 2010/081988

(56) Documents cités:
- WO-A-2007/144665
- WO-A-2009/003157
- SU-A1- 709 537

## Description

### DOMAINE DE L'INVENTION

L'invention a pour objet un procédé de préparation des hydrofluoropropènes.

### ARRIERE-PLAN TECHNOLOGIQUE

Les hydrofluorocarbones (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

Le 1,2,3,3,3- pentafluoropropène (HFO-1225ye) est un intermédiaire de synthèse dans la fabrication du 2,3,3,3-tétrafluoro-1-propène (HFO-1234yf).

La plupart des procédés de fabrication des hydrofluorooléfines font appel à une réaction de déhydrohalogénation. Ainsi, le document WO 03/027051 décrit un procédé de fabrication de fluorooléfines de formule CF₃CY=CXₙHₚ, dans laquelle X et Y représentent chacun un atome d'hydrogène ou d'halogène choisi parmi le fluor, le chlore, le brome ou l'iode ; n et p sont des nombres entiers et peuvent indépendamment prendre la valeur zéro, 1 ou 2 à condition que (n + p) = 2, comprenant la mise en contact d'un composé de formule CF₃C(R¹ₐR²_{b})C(R³_{c}R⁴_{d}) avec R¹, R², R³ et R⁴ représentant indépendamment un atome d'hydrogène ou d'halogène choisi parmi le fluor, le chlore, le brome ou l'iode à condition qu'au moins un de R¹, R², R³ et R⁴ est un atome d'halogène et qu'au moins un atome d'hydrogène et un atome d'halogène sont situés sur des atomes de carbone adjacent ; a et b peuvent indépendamment prendre la valeur zéro, 1 ou 2 à condition que (a + b) = 2 ; c et d peuvent indépendamment prendre la valeur zéro, 1, 2 ou 3 à condition que (c + d) = 3, avec au moins un hydroxyde de métal alcalin en présence d'un catalyseur de transfert phase.

Ce document enseigne à l'exemple 2, qu'en l'absence d'un catalyseur de transfert de phase, il n'y a pas de réaction lorsque le 1,1,1,3,3-pentafluoropropane (HFC-245fa) est mis en contact avec une solution aqueuse de 50 % en poids d'hydroxyde de potassium (KOH) pendant 24 heures à température ambiante et sous pression.

Ce document enseigne en outre une température de réaction compris entre -20°C et 80°C.

Le document WO 2008/075017 illustre la réaction de déhydrofluoration du 1,1,1,2,3,3-hexafluoropropane (HFC-236ea) en 1,2,3,3,3-pentafluoropropène (HFO-1225ye) à 150°C en présence d'une solution aqueuse de 50 % en poids de KOH. En l'absence d'un catalyseur de transfert de phase, la conversion au bout de 3 heures et demie est de 57,8 % et la sélectivité en HFO-1225ye est de 52,4 % (essai 1). En présence d'un catalyseur de transfert de phase, cette conversion est atteinte au bout de 2,5 heures seulement et la sélectivité est pratiquement inchangée (essai 4). Comme indiqué au tableau 2 de ce document pour accroître la sélectivité en HFO-1225ye, il est nécessaire d'utiliser un solvant organique.

WO 2007/056194 décrit la préparation de HFO-1234yf par déhydrofluoration du 1,1,1,2,3-pentafluoropropane (HFC-245eb) soit avec une solution aqueuse de KOH soit en phase gazeuse en présence d'un catalyseur, notamment sur catalyseur à base de nickel, de carbone ou une combinaison de ceux-ci.

Le document Knunyants et al, Journal of the USSR Academy of Sciences, Chemistry Department, "reactions of fluoro-olefins", report 13., "catalytic hydrogenation of perfluoro-olefins", 1960, décrit de façon distincte diverses réactions chimiques sur des composes fluorés. Ce document décrit la déhydrofluoration du 1,1,1,2,3,3-hexafluoropropane (236ea) par passage au travers d'une suspension de KOH en poudre dans l'éther de dibutyle, pour produire du 1,2,3,3,3-pentafluoropropène-1 (HFO-1225ye) avec un rendement de 60 % seulement. Ce document décrit également la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement.

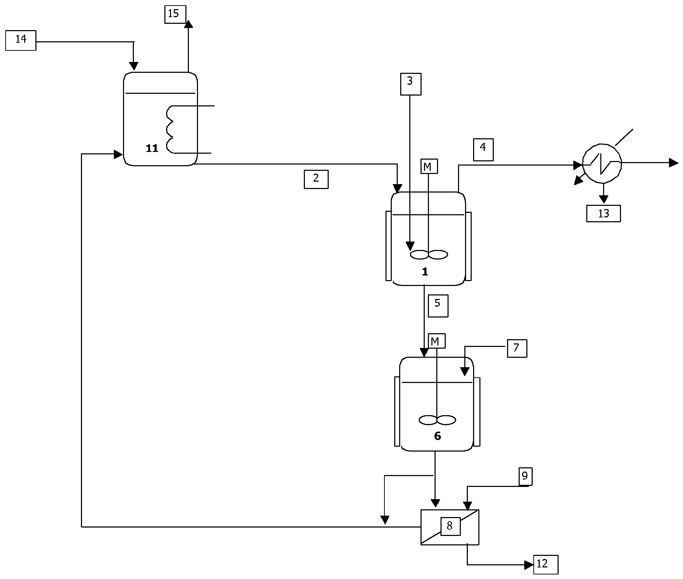

Par ailleurs, la figure 2 à la page 51 du deuxième fascicule du nouveau traité de chimie minérale de P. Pascal, Ed. 1963, montre l'allure des équilibres liquide solide du système eau et hydroxyde de potassium et les mesures sont rassemblées dans le tableau à la page 52. WO2009 003157 décrit un procédé de préparation d'hydrofluorooléfines en deux étapes, comprenant une fluoration en phase liquide d'hydrochloropropane en hydrofluoropropane suivie par une déhydrofluoration de l'hydrofluoropropane en hydrofluorooléfine.

Les réactions de déhydrofluoration telles que décrites ci-dessus conduisent, outre au composé hydrofluorooléfinique recherché, à la formation de l'eau et de fluorure de potassium. Par ailleurs, la mise en oeuvre d'une telle réaction en continu n'est pas aisée à l'échelle industrielle car au moins trois phases (gazeuse, liquide et solide) sont mises enjeu.

La présente invention fournit un procédé de fabrication continu ou semi-continu d'un composé (hydro)fluorooléfinique permettant de remédier aux inconvénients précités.

La présente invention a donc pour objet un procédé de fabrication continu ou semi-continu d'un composé (hydro)fluorooléfinique de formule (Ia)

CF₃-CF=CHZ (Ia)

dans laquelle Z représente un atome d'hydrogène ou fluor comprenant (i) la mise en contact d'un composé de formule CF₃CFRCHR'Z, dans laquelle Z a la même signification que dans la formule (Ia) et R représente un atome de fluor lorsque R' représente un atome d'hydrogène ou R représente un atome d'hydrogène lorsque R' représente un atome de fluor, avec de l'hydroxyde de potassium dans un réacteur agité, contenant un milieu réactionnel aqueux, muni d'au moins une entrée pour les réactifs et d'au moins une sortie, pour donner le composé (hydro)fluorooléfinique, qui est séparé du milieu réactionnel sous forme gazeux, et du fluorure de potassium, (ii) la mise en contact en milieu aqueux du fluorure de potassium formé en (i) avec de l'hydroxyde de calcium pour donner de l'hydroxyde de potassium et précipiter du fluorure de calcium, (iii) séparation du fluorure de calcium précipité à l'étape (ii) du milieu réactionnel et (iv) le milieu réactionnel est recyclé après éventuel ajustement de la concentration en hydroxyde de potassium à l'étape (i) ; caractérisé en ce l'hydroxyde de potassium représente entre 55 et 90 % en poids par rapport au poids du mélange eau et KOH présent dans le milieu réactionnel aqueux.

La présente invention permet ainsi d'obtenir un procédé avantageux car d'une part, l'hydroxyde de potassium est plus réactif que l'hydroxyde de calcium dans la réaction de déhydrofluoration et d'autre part, le fluorure de calcium est un sous produit valorisable.

Ainsi, le 2,3,3,3-tétrafluoropropène peut être obtenu par déhydrofluoration du 1,2,3,3,3-pentafluoropropane avec le KOH et/ou 1,2,3,3,3-pentafluoropropène par déhydrofluoration du 1,1,1,2,3,3-hexafluoropropane avec le KOH. Le 1,2,3,3,3-pentafluoropropène peut être sous la forme d'isomère cis et/ou trans.

La présente invention peut en outre être utilisée pour la fabrication du 1,3,3,3-tetrafluoropropène par déhydrofluoration du 1,1,3,3,3-pentafluoropropane avec le KOH.

Dans la suite du texte, les bornes des gammes de concentration et de température donnée sont incluses dans lesdites gammes.

Dans l'étape (i) du procédé selon la présente invention l'hydroxyde de potassium peut représenter entre 55 et 75 % en poids par rapport au poids du mélange eau et KOH présent dans le milieu réactionnel aqueux. Suivant la teneur, l'hydroxyde de potassium peut être sous forme de solution aqueuse ou à l'état fondu. Cette teneur élevée en KOH entraîne une augmentation du taux de conversion de l'hydrofluoroalcane en hydrofluoroalcène. En outre, du fait de ce milieu concentré en KOH, l'HF formé en (i) réagit immédiatement avec KOH pour former du KF moins corrosif que l'HF, ce qui permet d'utiliser en aval du réacteur de déshydrofluoration des réacteurs en acier carbone de faible coût comparer à des réacteurs en matériau noble (UB6 ou Inconel) pour le réacteur de déshydrofluoration. En outre, le « piégeage » de l'HF sous forme de KF facilite la séparation des différents produits entre eux (l'HF ayant tendance à former des azéotropes avec les hydrofluoroalcanes et hydrofluoroalcènes) ; ainsi une simple distillation est suffisante pour séparer les produits entre eux.

L'étape (i) est en général mise en oeuvre à une température telle que l'eau formée en cours de réaction de déhydrofluoration est éliminée, en partie ou en totalité, du milieu réactionnel par entraînement du flux gazeux comprenant le composé (hydro)fluorooléfinique, issu du réacteur agité. Cette température est de préférence comprise entre 80 et 180° C, avantageusement comprise entre 125 et 180°C, et tout particulièrement comprise entre 145 et 165° C. L'évaporation de l'eau lors de l'étape (i) va dans le sens de l'augmentation du taux de conversion l'hydrofluoroalcane en hydrofluoroalcène.

La réaction de déhydrofluoration de l'étape (i) peut être mise en oeuvre à pression atmosphérique mais on préfère travailler à une pression supérieure à la pression atmosphérique. Avantageusement, cette pression est comprise entre 1,1 et 2,5 bars.

La réaction de l'étape (ii) peut être mise en oeuvre dans un réacteur agité ou à lit fluidisé en faisant réagir de l'hydroxyde de calcium, de préférence en suspension dans l'eau, avec le fluorure de potassium en provenance de l'étape (i). La température de réaction peut varier dans des larges limites mais pour des raisons économiques, elle est de préférence comprise entre 50 et 150 ° C, par exemple de 75°C à 120° C et avantageusement entre 90 et 120° C.

Lorsqu'une suspension d'hydroxyde de calcium est utilisée à l'étape (ii), l'hydroxyde de calcium représente entre 2 et 40 % en poids par rapport au poids de la suspension.

Avantageusement, l'étape (ii) est mise en oeuvre dans le milieu réactionnel provenant de l'étape (i) comprenant de l'eau, de l'hydroxyde de potassium et du fluorure de potassium. Le fluorure de potassium en provenance de l'étape (i) et alimentant l'étape (ii) peut être dissous ou en suspension.

L'hydroxyde de potassium représente dans le milieu réactionnel de l'étape (ii) de préférence entre 2 et 50 % en poids par rapport au poids du mélange eau et hydroxyde de potassium du milieu.

Lorsque les étapes (i) et (ii) sont mises en oeuvre dans des réacteurs séparés, on peut prévoir une étape de dilution du milieu réactionnel entre l'étape (i) et l'étape (ii).

Le fluorure de calcium précipité à l'étape (ii) est séparé du milieu réactionnel, par exemple par filtration et/ou décantation. Préalablement à la filtration, on peut prévoir une étape de décantation. Le fluorure de calcium ainsi séparé est ensuite lavé à l'eau.

Au cours de l'étape de décantation, on peut prévoir le recyclage d'une partie de la suspension concentrée en fluorure de calcium à l'étape (ii). Avantageusement, le taux de solides de fluorure de calcium présent dans le milieu réactionnel de l'étape (ii) est compris entre 5 et 40 % en poids.

Après séparation du fluorure de calcium, le milieu réactionnel avec ou sans eaux de lavage du fluorure de calcium peut être recyclé à l'étape (i) après éventuel ajustement de la teneur en hydroxyde de potassium.

Selon un mode de réalisation de l'invention, les étapes (i) et (ii) peuvent être mises en oeuvre dans le même réacteur.

Il peut être avantageux d'utiliser un gaz inerte, de préférence de l'azote ou de l'hydrogène dans l'étape de déshydrofluoration.

Le procédé selon la présente invention a l'avantage de conduire à des rendements élevés même en l'absence de catalyseur de transfert de phase et/ou de solvant organique.

La présente invention comprend également les combinaisons des formes préférées quel que soit le mode de réalisation.

### PARTIE EXPERIMENTALE

### Exemple 1

La figure 1 donne le schéma d'un mode de réalisation de la présente invention. Un réacteur agité (1), en Nickel, équipé d'un dispositif de chauffage et de mesure de température du milieu réactionnel, contenant un mélange d'eau et de KOH, est alimenté en continu par une solution de KOH fondu (2) dans lequel le KOH est présent à 60 % en poids dans l'eau, et par du 1,1,1,2,3,3-hexafluoropropane (3). La température est maintenue à 160°C et la pression dans le réacteur est de 1,2 bars absolus. Les produits gazeux sortent du réacteur par un orifice (4) situé sur le couvercle du réacteur et l'eau contenue dans le flux gazeux est éliminée par condensation (13).

La sortie (5) du réacteur (1) est reliée à l'entrée du réacteur agité (6) et assure donc l'alimentation du réacteur (6) en hydroxyde de potassium, pouvant être en suspension dans le milieu aqueux. Une suspension de 10 % en poids d'hydroxyde de calcium dans l'eau est introduite dans le réacteur (6) par la voie (7). Le réacteur (6) est maintenu à une température comprise entre 100 et 120° C.

La sortie du réacteur (6) est reliée à un filtre (8) pour séparer le fluorure de calcium du milieu réactionnel, puis le laver à l'eau introduite par la voie (9) ; le milieu aqueux séparé du fluorure de calcium ainsi que les eaux de lavage du fluorure de calcium sont ensuite recyclés vers le réacteur (1) après ajustement de la concentration du KOH ; le fluorure de calcium est récupéré par la voie (12).

Le mélange de KOH fondu alimentant le réacteur (1) est préparé par chauffage (11) d'une solution aqueuse de 50 % en poids de KOH introduite par la voie (14) en vue d'une évaporation (élimination d'eau (15)).

### Exemple 2

On opère comme à l'exemple 1 sauf que l'on alimente le réacteur (1) en continu par du 1,2,3,3,3-pentafluoropropane à la place du 1,1,1,2,3,3-hexafluoropropane.

En utilisant une teneur en KOH supérieur à celle de l'art antérieur, on obtient des taux de conversion de l'hydrofluoroalcane en hydrofluoroalcène améliorée (donc une meilleure productivité), un produit valorisable le CaF2 et des couts de fabrication plus faible de l'hydrofluoroalcène.

## Revendications

1. Procédé de fabrication continu ou semi-continu d'un composé (hydro)fluorooléfinique de formule (Ia)
CF₃-CF=CHZ (Ia)
dans laquelle Z représente un atome d'hydrogène ou fluor comprenant (i) la mise en contact d'un composé de formule CF₃CFRCHR'Z, dans laquelle Z a la même signification que dans la formule (Ia) et R représente un atome de fluor lorsque R' représente un atome d'hydrogène ou R représente un atome d'hydrogène lorsque R' représente un atome de fluor, avec de l'hydroxyde de potassium dans un réacteur agité, contenant un milieu réactionnel aqueux, muni d'au moins une entrée pour les réactifs et d'au moins une sortie, pour donner le composé (hydro)fluorooléfinique, qui est séparé du milieu réactionnel sous forme gazeux, et du fluorure de potassium, (ii) la mise en contact en milieu aqueux du fluorure de potassium formé en (i) avec de l'hydroxyde de calcium pour donner de l'hydroxyde de potassium et précipiter du fluorure de calcium, (iii) séparation du fluorure de calcium précipité à l'étape (ii) du milieu réactionnel et (iv) le milieu réactionnel est recyclé après éventuel ajustement de la concentration en hydroxyde de potassium à l'étape (i) ; caractérisé en ce l'hydroxyde de potassium représente entre 55 et 90 % en poids par rapport au poids du mélange eau et KOH présent dans le milieu réactionnel aqueux.

2. Procédé selon la revendication 1, **caractérisé en ce que** le 2,3,3,3-tétrafluoropropène est obtenu par la mise en contact du 1,2,3,3,3-pentafluoropropane avec de l'hydroxyde de potassium et/ou le 1,2,3,3,3-pentafluoropropène par la mise en contact du 1,1,1,2,3,3-hexafluoropropane avec de l'hydroxyde de potassium.

3. Procédé selon l'une quelconque des revendications précédentes, **caractérisé en ce que** l'hydroxyde de potassium représente entre 55 et 75 % en poids.

4. Procédé selon l'une quelconque des revendications précédentes, **caractérisé en ce que** la température de mise en oeuvre de l'étape (i) est comprise entre 80 et 180° C.

5. Procédé selon l'une quelconque des revendications précédentes, **caractérisé en ce que** la température de l'étape (ii) est comprise entre 50 et 150 ° C.

6. Procédé selon l'une quelconque des revendications précédentes, **caractérisé en ce que** l'étape (ii) est mise en oeuvre dans le milieu réactionnel en provenance de l'étape (i).

7. Procédé selon l'une quelconque des revendications précédentes, **caractérisé en ce que** l'hydroxyde de potassium représente dans le milieu réactionnel de l'étape (ii) entre 2 et 50 % en poids par rapport au poids du mélange eau et hydroxyde de potassium du milieu.

8. Procédé selon l'une quelconque des revendications précédentes, **caractérisé en ce que** le fluorure de calcium à l'étape (iii) est filtré après une éventuelle étape de décantation.

9. Procédé selon la revendication 8, **caractérisé en ce que** lors de décantation une partie de la suspension concentrée en fluorure de calcium est recyclé à l'étape (ii).

## Patentansprüche

1. Verfahren zur kontinuierlichen oder halbkontinuierlichen Herstellung einer (Hydro)fluorolefin-Verbindung der Formel (Ia):
CF₃-CF=CHZ (Ia)
in der Z für ein Wasserstoff- oder Fluoratom steht, umfassend (i) Inkontaktbringen einer Verbindung der Formel CF₃CFRCHR'Z, in der Z die gleiche Bedeutung wie in Formel (Ia) hat und R für ein Fluoratom steht, wenn R' für ein Wasserstoffatom steht, oder R für ein Wasserstoffatom steht, wenn R' für ein Fluoratom steht, mit Kaliumhydroxid in einem ein wässriges Reaktionsmedium enthaltenden Rührreaktor mit mindestens einem Eingang für die Reaktanten und mindestens einem Ausgang zur Bildung der (Hydro)fluorolefin-Verbindung, die in gasförmiger Form von dem Reaktionsmedium abgetrennt wird, und von Kaliumfluorid, (ii) Inkontaktbringen des in (i) gebildeten Kaliumfluorids in einem wässrigen Medium mit Calciumhydroxid zur Bildung von Kaliumhydroxid und Ausfällung von Calciumfluorid, (iii) Abtrennen des in Schritt (ii) ausgefällten Calciumfluorids von dem Reaktionsmedium und (iv) Rezyklieren des Reaktionsmediums nach eventueller Einstellung der Kaliumhydroxidkonzentration zu Schritt (i); **dadurch gekennzeichnet, dass** das Kaliumhydroxid zwischen 55 und 90 Gew.-%, bezogen auf das Gewicht der in dem wässrigen Reaktionsmedium vorliegenden Mischung von Wasser und KOH, ausmacht.

2. Verfahren nach Anspruch 1, **dadurch gekennzeichnet, dass** 2,3,3,3-Tetrafluorpropen durch Inkontaktbringen von 1,2,3,3,3-Pentafluorpropan mit Kaliumhydroxid erhalten wird und/oder 1,2,3,3,3-Pentafluorpropen durch Inkontaktbringen von 1,1,1,2,3,3-Hexafluorpropan mit Kaliumhydroxid erhalten wird.

3. Verfahren nach einem der vorhergehenden Ansprüche, **dadurch gekennzeichnet, dass** das Kaliumhydroxid zwischen 55 und 75 Gew.-% ausmacht.

4. Verfahren nach einem der vorhergehenden Ansprüche, **dadurch gekennzeichnet, dass** die Temperatur der Durchführung von Schritt (i) zwischen 80 und 180 °C liegt.

5. Verfahren nach einem der vorhergehenden Ansprüche, **dadurch gekennzeichnet, dass** die Temperatur von Schritt (ii) zwischen 50 und 150 °C liegt.

6. Verfahren nach einem der vorhergehenden Ansprüche, **dadurch gekennzeichnet, dass** Schritt (ii) in dem aus Schritt (i) stammenden Reaktionsmedium durchgeführt wird.

7. Verfahren nach einem der vorhergehenden Ansprüche, **dadurch gekennzeichnet, dass** das Kaliumhydroxid in dem Reaktionsmedium von Schritt (ii) zwischen 2 und 50 Gew.-%, bezogen auf das Gewicht der Mischung von Wasser und Kaliumhydroxid des Mediums, ausmacht.

8. Verfahren nach einem der vorhergehenden Ansprüche, **dadurch gekennzeichnet, dass** das Calciumfluorid in Schritt (iii) nach einem fakultativen Dekantierungsschritt abfiltriert wird.

9. Verfahren nach Anspruch 8, **dadurch gekennzeichnet, dass** beim Dekantieren ein Teil der konzentrierten Calciumfluoridsuspension zu Schritt (ii) rezykliert wird.

## Claims

1. Process for the continuous or semi-continuous manufacture of a (hydro)fluoroolefin compound of formula (Ia):
CF₃-CF=CHZ (Ia)
in which Z represents a hydrogen or fluorine atom, comprising (i) bringing a compound of formula CF₃CFRCHR'Z, in which Z has the same meaning as in formula (Ia) and R represents a fluorine atom when R' represents a hydrogen atom or R represents a hydrogen atom when R' represents a fluorine atom, into contact with potassium hydroxide in a stirred reactor, containing an aqueous reaction medium, equipped with at least one inlet for the reactants and with at least one outlet, in order to give the (hydro)fluoroolefin compound, which is separated from the reaction medium in gaseous form, and potassium fluoride, (ii) bringing the potassium fluoride formed in (i) into contact, in an aqueous medium, with calcium hydroxide in order to give potassium hydroxide and to precipitate calcium fluoride, (iii) separation of the calcium fluoride precipitated in step (ii) from the reaction medium and (iv) the reaction medium is recycled after optional adjustment of the potassium hydroxide concentration to step (i); **characterized in that** the potassium hydroxide represents between 55% and 90% by weight relative to the weight of the water and KOH mixture present in the aqueous reaction medium.

2. Process according to Claim 1, **characterized in that** 2,3,3,3-tetrafluoropropene is obtained by bringing 1,2,3,3,3-pentafluoropropane into contact with potassium hydroxide and/or 1,2,3,3,3-pentafluoropropene is obtained by bringing 1,1,1,2,3,3-hexafluoropropane into contact with potassium hydroxide.

3. Process according to either one of the preceding claims, **characterized in that** the potassium hydroxide represents between 55% and 75% by weight.

4. Process according to any one of the preceding claims, **characterized in that** the operating temperature of step (i) is between 80°C and 180°C.

5. Process according to any one of the preceding claims, **characterized in that** the temperature of step (ii) is between 50°C and 150°C.

6. Process according to any one of the preceding claims, **characterized in that** step (ii) is carried out in the reaction medium originating from step (i).

7. Process according to any one of the preceding claims, **characterized in that** the potassium hydroxide represents, in the reaction medium of step (ii) between 2% and 50% by weight relative to the weight of the water and potassium hydroxide mixture of the medium.

8. Process according to any one of the preceding claims, **characterized in that** the calcium fluoride in step (iii) is filtered after an optional settling step.

9. Process according to Claim 8, **characterized in that** during settling, a portion of the suspension concentrated in calcium fluoride is recycled to step (ii).
